(19) Europäisches Patentamt
European Patent Office
Office européen des brevets

(11) Publication number: 0 007 930 B1

(12) **EUROPEAN PATENT SPECIFICATION**

(45) Date of publication of patent specification: **30.03.83**

(51) Int. Cl.[3]: C 07 D 237/20, A 61 K 31/50

(21) Application number: **78300341.1**

(22) Date of filing: **04.09.78**

# (54) Arylpyridazinylhydrazine salt, processes for preparing it and pharmaceutical compositions containing it.

(30) Priority: **04.08.78 GB 3231278**

(43) Date of publication of application: **20.02.80 Bulletin 80/4**

(45) Publication of the grant of the patent: **30.03.83 Bulletin 83/13**

(84) Designated Contracting States: **BE CH DE FR GB LU NL SE**

(56) References cited: **FR - A - 2 275 213**

(73) Proprietor: **SMITH KLINE & FRENCH LABORATORIES LIMITED**
**Mundells**
**Welwyn Garden City Hertfordshire, AL7 1EY (GB)**

(72) Inventor: **Burpitt, Brian Eric**
**36 Brook Drive**
**Stevenage Hertfordshire (GB)**
Inventor: **Graham, Michael James**
**3 Stoneycroft**
**Welwyn Garden City Hertfordshire (GB)**
Inventor: **Roe, Anthony Maitland**
**10 Lodge Drive**
**Hatfield Hertfordshire (GB)**

(74) Representative: **Hargreaves, Gerald Henry, Dr. et al,**
**P.O.Box 39**
**Welwyn Garden City Hertfordshire AL7 1EY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 007 930 B1

# Arylpyridazinylhydrazine salt, processes for preparing it and pharmaceutical compositions containing it

This invention relates to an arylpyridazinylhydrazine salt, pharmaceutical compositions containing it, and processes for preparing the salt and the compositions.

Belgian Patent 830,158 an equivalent of FR—A—2275273 describes a class of arylpyridazinylhydrazine compounds which have the basic structure (I):

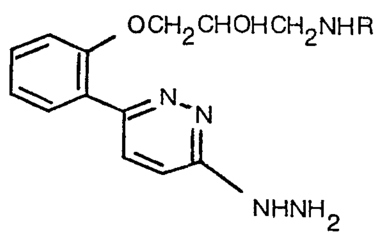

I

in which the rings can be substituted with a variety of named substituents, and R is isopropyl, tertiary butyl or phenylethyl, and which exhibit both $\beta$-adrenergic blocking and vasodilator activity. In these compounds the carbon atom to which the hydroxy group is attached is asymmetric, so that by normal methods of synthesis they are obtained as the racemates consisting of equal amounts of two optical isomers. Among the specific compounds particularly described is the compound of structure I where R is tertiary butyl, namely 3 - [2 - (3 - t - butylamino - 2 - hydroxypropoxy)phenyl] - 6 - hydrazinopyridazine (II), the preparation of which is given in Example 2 of Belgian Patent 830,158. The present invention is concerned with a special form of this compound. Belgian Patent 830,158 states that for therapeutic use the compounds of the class concerned can be employed in pharmaceutical compositions either in their basic form of in the form of an addition salt with a pharmaceutically acceptable acid, such salts including those with hydrochloric, hydrobromic, hydriodic, sulphuric, acetic, citric and maleic acids. The only form described for compound (II) is that shown in Example 2 of Belgian Patent 830,158 namely the hemisulphate hemihydrate, and other specific compounds are disclosed as prepared in the form of an amorphous citrate, a sulphate, or the free base.

Compound (II) and the other compounds of Belgian Patent 830,158 have three basic structural features, namely a hydrazino group, a linked pair of heterocyclic nitrogen atoms and a secondary amino group, and are capable of giving rise to mono-, di- and tri-salts of monobasic acids, and corresponding salts of dibasic acids, including the hemisulphate referred to. The compounds also show a facility for forming salts which in the solid crystalline state contain solvent of crystallisation, as in the hemisulphate hemihydrate of compound (II).

A specific form of salt of the racemate of compound (II) has now been discovered that has valuable advantages for pharmaceutical formulation and medicinal effect that are not shown by many other salts which have also been investigated.

It has been found that the racemate of compound (II) forms a crystalline dihydrochloride monohydrate which is a particularly stable, non-hygroscopic solid well-suited to tabletting and which can be consistently recrystallised with good volume efficiency from suitable solvents: this crystalline solid contains about 1.1 mols (4.5% by weight) water of crystallisation, and analyses by either the Karl Fischer method or by a thermogravimetric method (Perkin-Elmer TGS/1 thermobalance) give consistent results corresponding to this water content. In contrast, the solid hemisulphate hemihydrate of the racemate of compound (II) tends to deteriorate on standing, whether because of hygroscopicity or for some other reason: furthermore the amount of solvent of crystallisation of this hemisulphate shows a water content after crystallisation under standard conditions which varies unpredictably. Therefore the hemisulphate salt form is not convenient for use in the commercial preparation of solid oral dosage forms such as tablets and powder filled capsules. Furthermore, the dihydrochloride gives aqueous solutions that are more stable than those of the hemisulphate. It has also been found that the oral administration to mammals in high doses of the dihydrochloride monohydrate is much less liable to cause diarrhoea than when the hemisulphate hemihydrate is administered in corresponding doses.

Of other salts of the racemate of compound (II) which have been investigated, the monohydrochloride was an uncrystallisable oil, the trihydrochloride was obtained as crystals which were difficult to recrystallise and its aqueous solutions are less suitable for intravenous administration because they are highly acidic, the dihydrobromide was an oil which crystallised on standing and gave poor analytical data, the dihydriodide could not be recrystallised to give a sample which had satisfactory analytical data, and the salts prepared from one molar equivalent amounts of citric and maleic acids and one and two molar equivalent amounts of acetic acid could not be crystallised. Salts prepared from many other pharmaceutically-acceptable acids, for example the diprotonated salts formed with orthophosphoric, palmitic, stearic, benzoic, succinic, fumaric, malonic, glutaric, itaconic, glycollic, D,L-malic, L(—)tartaric, aspartic and pamoic acids were all oils which could not by crystallised.

The invention therefore provides an acid

addition salt of racemic 3 - [2 - (3 - t - butylamino - 2 - hydroxypropoxy)phenyl - 6 - hydrazinopyridazine characterised in that the salt is the dihydrochloride monohydrate.

The dihydrochloride monohydrate forms colourless needles whose melting point is unreliable as a physical property for characterisation, because of dehydrative decomposition and a transition from solid to liquid phase that is not clearly defined. In melting point determinations in unsealed capillary tubes using a starting temperature of 130°C and raising it by 3°C per minute the compound may melt over several degrees at any temperature in the range of from 154°C to 168°C. When the compound is subjected to differential scanning calorimetric thermogravimetric analysis (DSC-TGA) in an open container with the temperature increasing at 10°C per minute two transition temperature ranges are observed, 110—145°C during which there is a weight loss of from 4.2 to 4.3% and 150—180°C with a weight loss of 0.2%: melting occurs during this second weight loss normally in the range 154—159°C. The weight loss calculated for 1 molar proportion of water is 4.27% and the total weight loss is consistently higher than this at about 4.5%. When the dihydrochloride monohydrate is subjected to DSC-TGA in a sealed container it appears to melt at 143°C.

When a 5 mg sample of the dihydrochloride monohydrate was heated at 100°C for 1.5 hours in a DSC-TGA apparatus a weight loss of 4.31% was recorded. The sample was allowed to cool to room temperature, exposed to normal atmosphere for 15 minutes and reanalysed. When the apparatus was programmed to scan from 40°C to 180°C at 10°C per minute a weight loss of 3.89% occurred over the range of 110—145°C. This indicates that the monohydrate is dehydrated on heating and the anhydrous dihydrochloride reforms the monohydrate on cooling by absorbing water from moist air.

When 15 mg samples of the dihydrochloride monohydrate were (a) stored at 40°C for 16 hours, (b) stored at room temperature over phosphorus pentoxide for 16 hours, (c) stored at room temperature and 0.5 mm Hg over phosphorus pentoxide for 16 hours, there were negligible (less than 0.3%) losses in weight. When 0.6 g samples of the dihydrochloride monohydrate were stored at 22°C and 90% humidity for 3 weeks the samples showed a 0.7% gain in weight. Thus, it appears that the dihydrochloride monohydrate is a stable form over normal conditions of temperature and humidity and is suitable for solid pharmaceutical compositions.

Methods for the preparation of compound (II) as the free base and its acid addition salts are described in Belgian Patent 830,158.

A process of the invention is one for preparing racemic 3 - [2 - (3 - t - butylamino - 2 - hydroxypropoxy)phenyl] - 6 - hydrazinopyridazine dihydrochloride monohydrate in which a solution of racemic 3 - [2 - (3 - t - butylamino - 2 - hydroxypropoxy)phenyl] - 6 - hydrazinopyridazine or an acid addition salt of this compound other than the dihydrochloride is reacted with hydrogen chloride and water in the amounts required to produce the dihydrochloride monohydrate, and the dihydrochloride monohydrate is isolated as the solid particulate salt. The hydrogen chloride required is the stoichiometric amount for provision of two molar equivalents of hydrogen chloride for each molar equivalent of pyridazine base, though more or less can be used, for instance as little as 1.8 and as much as 2.2 molar equivalents if loss of material as monohydrochloride or trihydrochloride during the isolation step is acceptable. Where an aqueous solution of the dihydrochloride is prepared as an initial step, the required amount of hydrochloric acid is that needed to produce a solution of pH from 3 to 4 depending on the concentration. However an excess of water to produce the monohydrate can be introduced and the excess eliminated during isolation.

Preferably a solution of the hydrazinopyridazine in a liquid medium containing the required hydrogen chloride and water is caused to precipitate solid particles of dihydrochloride monohydrate, and the particles are separated off. The liquid medium can be water or an organic solvent, for example dichloromethane, ethanol, n-propanol, isopropyl alcohol or t-butanol. The precipitation of solid particles can be effected directly from the liquid medium or addition of hydrochloric acid or water, as when hydrochloric acid is added to a solution of the pyridazine base in dichloromethane, or water is added to a solution of the anhydrous dihydrochloride in n-propanol. Precipitation can also be effected by cooling a solution of the dihydrochloride monohydrate, for instance a solution in n-propanol or t-butanol containing a small excess of water, for example n-propanol containing 7—9% by weight of water. Precipitation can be effected by removing solvent in the vapour phase, as for instance by evaporation under reduced pressure, or by adding a diluent which reduces the solubility of the dihydrochloride monohydrate in the liquid medium, for example tetrahydrofuran, ether or toluene.

The separated particles of product are preferably freed from traces of solvent (including an excess of water) by heating, for instance with the particles as a fluid bed using a current of air at 50—60°C, or by heating under reduced pressure, for instance at 0.5 mm Hg at 90°C. Such treatments may cause the loss of some or all of the water of hydration, and where water of hydration is lost the product is allowed to rehydrate on cooling, which can be effected simply by allowing access of moist air.

The hydrogen chloride can be provided by adding hydrogen chloride in aqueous or nonaqueous form and by exchange of anions using

a different salt of compound (II). Exchange of anions can be achieved by contacting a solution of a salt of compound (II) with an excess of an anion-exchange resin in the chloride form or by adding a soluble chloride salt of a metal which forms an insoluble salt with a different anion originally associated with compound (II); for example the hemisulphate of compound (II) can be converted into the dihydrochloride by adding a concentrated solution of one molar equivalent of calcium chloride to a solution of two molar equivalents of the hemisulphate of compound (II), filtering off insoluble calcium sulphate to give a solution of the monohydrochloride of compound (II), and by adding a molar equivalent of hydrochloric acid to give the dihydrochloride.

The invention also provides pharmaceutical compositions comprising the dihydrochloride monohydrate and a pharmaceutically-acceptable diluent or carrier and a process for making such pharmaceutical compositions by bringing the dihydrochloride monohydrate into association with the diluent or carrier. These compositions can be in solid or liquid form for oral administration or solutions for administration by intravenous injection. These pharmaceutical compositions are prepared in a conventional dosage unit form by incorporating racemic 3 - [2 - (3 - t - butylamino - 2 - hydroxypropoxy)phenyl] - 6 - hydrazinopyridazine dihydrochloride monohydrate in a nontoxic amount to produce an antihypertensive effect in hypertensive patients, with a pharmaceutically-acceptable diluent or carrier. Preferably the composition will contain 50 mg to 300 mg of active ingredient per dosage unit. The pharmaceutical can be made from the solid dihydrochloride monohydrate by (a) encapsulating, (b) mixing with solid diluent or carrier and shaping into dosage unit form, or (c) forming a dispersion in liquid diluent or carrier. The pharmaceutical compositions can be made by mixing, granulating and compressing solid ingredients to form tablets which can be spray-coated, or by mixing and dissolving the ingredients for a liquid preparation. Preferably the compositions are made by a process which does not involve contacting the wet ingredients with salts of transition metals and heavy metals, particularly iron salts.

The pharmaceutical carrier employed can be a solid or liquid. Examples of solid carriers are lactose, maize starch, potato starch, or modified starches, dicalcium phosphate, terra alba, sucrose, celluloses, talc, gelatin, agar, pectin, acacia, magnesium stearate and stearic acid. Examples of liquid carriers are syrup, peanut oil, olive oil, alcohol, propylene glycol, polyethylene glycols and water.

If a solid carrier is used, the composition can be prepared in the form of a tablet, capsule containing powder or pellets, troche or lozenge. The amount of solid carrier in a unit dosage form is generally from 25 mg to 300 mg. If a liquid carrier is used, the composition can be in the form of a syrup, emulsion, multiple emulsion, sterile injectable liquid or an aqueous or nonaqueous solution or liquid suspension. Other additives such as preservatives, for example, antioxidants or antibacterials, and/or flavouring or colouring agents can also be included. The sterile liquids can be prepared in ampoules, multidose vials or unit dose disposable systems.

The invention is illustrated by the following Examples in which temperatures are in °C.

Example 1

Racemic 3 - [2 - (3 - t - butylamino - 2 - hydroxypropoxy)phenyl] - 6 - chloropyridazine hydrochloride (10 g) was added with stirring to hydrazine hydrate (40 ml, 100%) and the mixture heated under reflux for 2 hours. On cooling to 30° the base 3 - [2 - (3 - t - butylamino - 2 - hydroxypropoxy)phenyl] - 6 - hydrazinopyridazine separated as an oil and the mixture was extracted with dichloromethane (3×10 ml), and the extracts repeatedly washed with small amounts of water until hydrazine was absent as shown by thin layer chromatography on silica gel using potassium iodoplatinate reagent to spray the plate developed with chloroform/methanol (4:1), when any hydrazine appears as a bleached halo around the blue spot due to the hydrazinopyridine.

In this way there was obtained a solution of the racemic hydrazinopyridazine base (8.93 g, 0.027 mol) in dichloromethane (30 ml). To this solution was added n-propanol (40 ml) and concentrated hydrochloric acid (4.45 ml, 0.054 mol) and the solution was distilled until the temperature of the vapour was 85° (to remove dichloromethane), water (2 ml) was added and the solution was allowed to cool to room temperature. Crystals of the dihydrochloride monohydrate which had formed during cooling were filtered off and heated in an oven under reduced pressure (0.5 mm Hg; $0.66 \cdot 10^{-3}$ bar) at 90° for 6 hours to remove solvent, after which they were placed in undried air for 4 hours and allowed to take up water sufficient to ensure complete reformation of the monohydrate (9.0 g).

Example 2

Crystals of the dihydrochloride monohydrate obtained by the procedure described in Example 1 using 200 g of racemic 3 - [2 - (3 - t - butylamino - 2 - hydroxypropoxy)phenyl] - 6 - chloropyridazine hydrochloride were dried in a fluid bed drier at 60° for 1.5 hours to give the dihydrochloride monohydrate (190 g).

Example 3

To a vigorously stirred and externally cooled solution of racemic hydrazinopyridazine base (789 g, 2.35 mol) in dichloromethane (3.0 litres), obtained by the procedure described in Example 1, was added concentrated hydrochloric acid (SG 1.18, 392 ml, 4.7 mol) during 10 min. After 1 hour stirring in an ice-water

bath the mixture was filtered to separate the precipitated solid dihydrochloride monohydrate. The collected undried solid was washed with dichloromethane and recrystallised from n-propanol (3.2 litres), the recrystallised monohydrate was then heated under reduced pressure at 90° for 6 hours to remove solvent, after which it was placed on trays in undried air at room temperature for 16 hours to ensure complete reformation of the monohydrate (800 g), m.p. 157—60°.

Example 4

To a solution of racemic hydrazinopyridazine base in dichloromethane (7.5 litres) obtained from racemic 3 - [2 - (3 - t - butylamino - 2 - hydroxypropoxy)phenyl] - 6 - chloropyridazine hydrochloride (1.98 kg) by the procedure described in Example 1, was added concentrated hydrochloric acid (SG 1.18, 0.912 litres) with external cooling. The solid which crystallised out on cooling was filtered off and was air dried at room temperature overnight. The partly dried material (2.7 kg) was suspended in n-propanol (16 litres) and the mixture was distilled until the head temperature reached 93°. In this way there was obtained a solution of 3 - [2 - (3 - t - butylamino - 2 - hydroxypropoxy)phenyl] - 6 - hydrazinopyridazine dihydrochloride (1.95 kg) in n-propanol (10 litres). To this filtered solution was added water (0.5 litre), the mixture was allowed to cool to room temperature and the solid which crystallised out was collected washed well with n-propanol and dried at 90° at 0.5 mm Hg pressure and placed on trays in undried air at room temperature for 16 hours to ensure complete reformation of the dihydrochloride monohydrate, (1.94 kg), m.p. 163—166°.

Example 5

A mixture of hydrazine hydrate (64% hydrazine, 131 kg) and racemic 3 - [2 - (3 - t - butylamino - 2 - hydroxypropoxy)phenyl] - 6 - chloropyridazine hydrochloride (30 kg) was heated under reflux for 2.5 hours and cooled to 20°. Dichloromethane (74 litres) was added, the mixture was stirred and the dichloromethane layer was separated off. The hydrazine phase was twice extracted with dichloromethane (20 and 16 litres) and the combined dichloromethane extracts were washed five times with water. Aqueous hydrochloric acid (SG 1.18, 15.7 kg) and n-propanol (120 litres) were added to the dichloromethane solution and the mixture was distilled until the vessel temperature was 93°. The mixture was cooled to 15—20°, stirred for 30 minutes and the crystalline solid was centrifuged out and washed with n-propanol. Solvent was removed at room temperature by placing the solid in a forced air oven for 36 hours.

The solid was then passed through a No. 6 mesh screen and dried in a fluid bed drier at 55° for 3 hours to give racemic 3 - [2 - (3 - t - butylamino - 2 - hydroxypropoxy)phenyl] - 6 - hydrazinopyridazine dihydrochloride monohydrate (27.2 kg).

Preferably the water content of the mixture is checked by the Karl Fischer method before the solution in n-propanol is allowed to cool, and if the water content is outside the range 7—9% by weight it is adjusted by adding water, or by adding n-propanol and distilling the mixture further.

Example 6

Racemic 6 - [2 - (3 - t - butylamino - 2 - hydroxypropoxy)phenyl] - 3 - (2H) - pyridazinethione (1 g) was added with stirring to hydrazine hydrate (4 ml) and heated under reflux for 1.5 hours. The solution was cooled to 25—30°, when the base 3 - [2 - (3 - t - butylamino - 2 - hydroxypropoxy)phenyl] - 6 - hydrazinopyridazine separated as an oil. Dichloromethane (10 ml) was added to dissolve the base and the organic layer which separated after mixing was isolated and washed with water (3x1 ml). The organic phase was dried over magnesium sulphate and the solvent was removed by evaporation to leave the base as a brown oil (0.98 g, 2.9 mmol). This oil was dissolved in ethanol (3 ml) and concentrated hydrochloric acid (SG 1.18, 0.48 ml, 5.8 mmol) and ether (3 ml) were added. The crystals were filtered off and dried by heating in a vacuum oven at 90° and 0.5 mm Hg ($0.66 \cdot 10^{-3}$ bar) for 16 hours, and they were then allowed to take up water from the atmosphere at room temperature sufficient to reform the dihydrochloride monohydrate, m.p. 156—64°.

Example 7

The racemic free base 3 - [2 - (3 - t - butylamino - 2 - hydroxypropoxy)phenyl] - 6 - hydrazinopyridazine obtained as an oil (4.8 g, 0.014 mol) by a procedure similar to that of Example 6 was dissolved in t-butanol (10 ml) at 60° and concentrated hydrochloric acid (SG 1.18, 2.4 ml, 0.024 mol) added, the mixture was heated to boiling and water added dropwise until solution was complete. The solution was allowed to cool to 0° overnight when crystals of the dihydrochloride monohydrate were deposited, filtered off and dried at 90° and 0.5 mm Hg ($0.66 \cdot 10^{-3}$ bar) pressure for 6 hours and exposed to undried air at room temperature to give the dihydrochloride monohydrate, (4.0 g), m.p. 167—70°.

Example 8

The racemic free base 3 - [2 - (3 - t - butylamino - 2 - hydroxypropoxy)phenyl] - 6 - hydrazinopyridazine obtained as an oil by a procedure similar to that of Example 6 was dissolved with warming in n-propanol (25 ml) and concentrated hydrochloric acid (SG 1.18, 2.52 ml, 0.025 mol) added; the solution obtained was cooled to 20° and the crystals deposited

were filtered off to give the dihydrochloride monohydrate, (3.5 g) m.p. 154—60°.

Example 9

Racemic 3 - [2 - (3 - t - butylamino - 2 - hydroxypropoxy)phenyl] - 6 - hydrazinopyridazine hemisulphate hemihydrate (100 mg) was dissolved in 0.1N hydrochloric acid (1.27 ml) and calcium chloride hexahydrate (30 mg) dissolved in the minimum water for solution at room temperature was added. Calcium sulphate was removed by filtration and the solution evaporated under reduced pressure to give a solid residue (101 mg) which was recrystallised from n-propanol and was left in contact with undried air to form the dihydrochloride monohydrate (66 mg).

Example 10

Racemic 3 - [2 - (3 - t - butylamino - 2 - hydroxypropoxy)phenyl] - 6 - hydrazinopyridazine hemisulphate hemihydrate (48.6 mg 0.123 mmol) in distilled water (10 ml) was run through a column of Amberlite IRA-400 (10 ml, in chloride form) and was eluted with distilled water (30 ml). Amberlite IRA-400 is a quaternary ammonium ion-exchange resin with a matrix of 8% cross-linked polystyrene-divinylbenzene (Amberlite is a registered trade mark). Hydrochloric acid (1.0N, 0.124 ml) was added to the eluate which was freeze-dried and the dried powder was further dried at 80° and 1 mm Hg for 20 hours. This solid was recrystallised from n-propanol containing 8% by weight of water, and the product was dried at 80° and 1 mm Hg ($1.33 \cdot 10^{-3}$ bar) for 8 hours and allowed to stand in undried air to give the dihydrochloride monohydrate which gave a 4.94% weight loss on DSC-TGA.

Example 11

A pharmaceutical composition in tablet form for oral administration is prepared by the following procedure. Racemic 3 - [2 - (3 - t - butylamino - 2 - hydroxypropoxy)phenyl] - 6 - hydrazinopyridazine dihydrochloride monohydrate (106 parts) is added to a mixture of microcrystalline cellulose (83 parts) and microfine silica (1 part) and mixed. Magnesium stearate (2 parts) and sodium glycollate-starch (10 parts) are mixed in. Half this mixture is granulated by compression, and screened through a No. 10 sieve and mixed with the remaining half of the mixture, mixed and compressed into tablets using 9.0 mm or 11.0 mm normal concave punches and dies. The tablets are warmed, film coated by spraying with a mixture of hydroxypropyl methylcellulose (60 g), ethylcellulose (15 g) and diethyl phthalate (9 g) and approved colourant in a mixture of methanol (1 litre) and dichloromethane (1 litre), and rolled with finely powdered carnauba wax. Use of 9.0 mm dies and punches gives tablets containing 127 mg of the dihydrochloride monohydrate and use of 11.0 mm dies and punches give tablets containing 254 mg of the dihydrochloride monohydrate.

Example 12

Pharmaceutical compositions for administration by intravenous injection are prepared by the following procedure. Racemic 3 - [2 - (3 - t - butylamino - 2 - hydroxypropoxy)phenyl] - 6 - hydrazinopyridazine dihydrochloride monohydrate is dissolved in freshly distilled water to give a solution containing 12.74 mg dihydrochloride monohydrate per ml and the solution passed through a 0.8 micron filter and filled under nitrogen into 5 ml flint glass ampoules. The ampoules are heat-sealed and sterilised at 115—116° for 30 minutes. Alternatively a solution containing 31.86 mg dihydrochloride monohydrate per ml is prepared and this is sealed into 2 ml ampoules.

Example 13

A pharmaceutical composition for oral administration is prepared by mixing racemic 3 - [2 - (3 - t - butylamino - 2 - hydroxypropoxy)phenyl] - 6 - hydrazinopyridazine dihydrochloride monohydrate (100 parts) with lactose (30 parts) and magnesium stearate (1 part), screening the mixture and filling No. 1 hardshell gelatin capsules with the mixture so that each capsule contains 254 mg of the dihydrochloride monohydrate.

**Claims**

1. Acid addition salt of racemic 3 - [2 - (3 - t - butylamino - 2 - hydroxypropoxy)phenyl] - 6 - hydrazinopyridazine characterised in that the salt is the dihydrochloride monohydrate.

2. A process for preparing the salt of Claim 1, characterised in that a solution of racemic 3 - [2 - (3 - t - butylamino - 2 - hydroxypropoxy)phenyl] - 6 - hydrazinopyridazine or an acid addition salt of this compound other than the dihydrochloride is reacted with hydrogen chloride and water in the amounts required to produce the dihydrochloride monohydrate, and the dihydrochloride monohydrate is isolated as the solid particulate salt.

3. A process according to Claim 2, characterised in that the dihydrochloride monohydrate is precipitated as solid particles directly from the liquid medium on addition of hydrochloric acid or water, or by cooling the solution and then the particles are separated off.

4. A process according to Claim 3 characterised in that the separated particles are freed from traces of solvent by heating, and where water of hydration is lost by heating the product is allowed to rehydrate on cooling.

5. A pharmaceutical composition comprising the salt of Claim 1 and a pharmaceutically-acceptable diluent or carrier.

6. A pharmaceutical composition when prepared from the salt of Claim 1 by encapsulating, mixing with solid diluent or carrier and shaping into dosage unit form, or forming a dispersion in liquid diluent or carrier.

**Patentansprüche**

1. Säureadditionssalz eines razemischen 3 - [2 - (3 - tert. - Butylamino - 2 - hydroxypropoxy) - phenyl] - 6 - hydrazinopyridazin, dadurch gekennzeichnet, daß das Salz das Dihydrochloridmonohydrat ist.

2. Ein Verfahren zur Herstellung des Salzes nach Anspruch 1, dadurch gekennzeichnet, daß man eine Lösung des razemischen 3 - [2 - (3 - tert. - butylamino - 2 - hydroxypropoxy) - phenyl] - 6 - hydrazinopyridazins oder eines Säureadditionssalzes dieser Verbindung, jedoch nicht das Dihydrochlorid, mit Chlorwasserstoff und Wasser in den Mengen umsetzt, die nötig sind, um das Dihydrochlorid-monohydrat herzustellen, und das Dihydrochlorid-monohydrat als Salz in Form von Feststoffteilchen isoliert.

3. Ein Verfahren nach Anspruch 2, dadurch gekennzeichnet, daß das Dihydrochlorid-monohydrat in Form von Feststoffteilchen direkt aus dem flüssigen Medium durch Zugabe von Salzsäure oder Wasser oder durch Kühlen der Lösung ausgefällt wird, wobei anschließend die Teilchen abgetrennt werden.

4. Ein Verfahren nach Anspruch 3, dadurch gekennzeichnet, daß die abgetrennten Teilchen von Lösungsmittelspuren durch Erhitzen befreit werden und daß man, falls beim Erhitzen Hydratwasser verlorengeht, eine Rehydratisierung des Produktes beim Abkühlen zuläßt.

5. Arzneimittel, enthaltend das Salz nach Anspruch 1 und ein pharmazeutisch verträgliches Verdünnungsmittel oder einen pharmazeutisch verträglichen Trägerstoff.

6. Arzneimittel, hergestellt aus dem Salz gemäß Anspruch 1 durch Einkapseln, Vermischen mit dem festen Verdünnungsmittel oder Trägerstoff und Anpassen in die Dosiseinheit oder durch Bildung einer Dispersion in einem flüssigen Verdünnungsmittel oder Trägermaterial.

**Revendications**

1. Sel d'addition d'acide de la 3 - [2 - (3 - t - butylamino - 2 - hydroxypropoxy) - phényl] - 6 - hydrazinopyridazine racémique caractérisé en ce que le sel est le dichlorhydrate monohydraté.

2. Procédé de préparation du sel de la revendication 1, caractérisé en ce que l'on fait réagir une solution de 3 - [2 - (3 - t - butylamino - 2 - hydroxypropoxy)phényl] - 6 - hydrazinopyridazine racémique ou d'un sel d'addition d'acide de ce composé autre que le dichlorhydrate avec de l'acide chlorhydrique et de l'eau dans les quantités requises pour produire le dichlorhydrate monohydraté et que l'on isole le dichlorhydrate monohydraté sous forme de sel solide particulaire.

3. Procédé suivant la revendication 2, caractérisé en ce que l'on précipite le dichlorhydrate monohydraté sous forme de particules solides directement à partir du milieu liquide par addition d'acide chlorhydrique ou d'eau, ou par refroidissement de la solution et que l'on isole ensuite les particules.

4. Procédé suivant la revendication 3, caractérisé en ce qu'on libère les particules séparées des traces de solvant par chauffage et que là où l'eau d'hydratation est perdue par chauffage, on laisse le produit se réhydrater sous refroidissement.

5. Composition pharmaceutique contenant le sel de la revendication 1 et un diluant ou excipient pharmaceutiquement acceptable.

6. Composition pharmaceutique lorsqu'elle est préparée à partir du sel de la revendication 1 par encapsulation, mélange avec un diluant ou excipient solide et façonnage en forme de dose unitaire ou formation d'une dispersion dans un diluant ou excipient liquide.